# EUROPEAN PATENT SPECIFICATION

(11) **EP 0 374 257 B1**
(45) Date of publication and mention of the grant of the patent: **31.08.1994**
(21) Application number: 89905176.7
(22) Date of filing: 02.05.1989
(51) Int. Cl.: A61K 37/66, A61K 47/10, A61K 47/14

(54) **STABLE INTERFERON $g(b) COMPOSITION**
STABILE INTERFERON-BETA-ZUSAMMENSETZUNG
COMPOSITION D'INTERFERON $g(b) STABLE

(30) Priority: 06.05.1988 JP 110921/88
(43) Date of publication of application: 27.06.1990
(73) Proprietor: TORAY INDUSTRIES, INC., Tokyo 103 (JP)
(72) Inventor: HARA, Michio, Kawasaki-shi Kanagawa 215 (JP); TANIGUCHI, Makoto, Meguro-ku Tokyo 152 (JP)
(74) Representative: Kador & Partner
(86) International application number: JP8900466
(87) International publication number: WO8910756

(56) References cited:
- EP-A- 0 152 345
- US-A- 4 483 849
- US-A- 4 605 556
- US-A- 4 710 376
- D4: EP-A-0 080 879
- DAICEL catalogue
- D5: US-A-4 605 555
- D6: Database WPIL/DERWENT, accession no. 87-230 754 (33), Derwent Publications Ltd., London, JP-A-62 153 226 (Toray Ind. Inc.)

## Description

### TECHNICAL FIELD

This invention relates to a stable gel or ointment formulation of interferon-β (IFN-β).

### BACKGROUND ART

The method of the mass production of IFN-β, which inhibits virus reproduction, was developed using cell culture and DNA recombinant technology. IFN-β produced by the former method is already commercially available. Although highly refined IFN-β is in demand as a pharmaceutical agent, there is a problem in that the more refined it is, the less stable it becomes. The uses of polyol (Japanese Patent Application Laid-Open (Kokai) No. 92619/83 and so on), human serum albumin (International Publication No. W083/01198), organic acid buffer (Japanese Patent Application Laid-Open (Kokai) No. 92621/83), carboxymethyl cellulose (Japanese Patent Application Laid-Open (Kokai) No. 153226/87) and so on have already been proposed as methods to increase the stability of IFN-β, and certain stabilizing effects have been achieved.

On the other hand, as with the IFN-β preparation, ointments which contain polyethylene glycol, propylene glycol, human serum albumin, polyvinyl pyrrolidone and methyl- or propyl-p-hydroxybenzoate as the base, are marketed by Serono (Italy) and Inter-Yeda (Israel), and are stated to be stable for 1 year when preserved at the low temperature of 4 to 8°C.

Although IFN-β can be stabilized by the above mentioned methods of the prior art, various additives such as viscosity-increasing agents or preservatives still need to be added to produce an ointment preparation of IFN-β. For example, when carboxymethyl cellulose "Daicel 2200" is added as the viscosity-increasing agent, IFN-β is remarkably less stable than when it is in 2 wt % of carboxymethyl cellulose ("Daicel 1240") - 50 wt % of glycerin - 48 wt % of 0.1 M citric acid buffer (pH 5) even if 4 kinds of IFN-β stabilizer such as 30 wt % or more (50 wt %, in this experiment) of glycerin, human serum albumin (International Patent Provisional Publication No. W083/01198), organic acid buffer (Japanese Patent Provisional Publication No. 92621/83) and 2 wt % of carboxymethyl cellulose ("Daicel 1240") (Japanese Patent Application Laid-Open (Kokai) No. 153226/77) are added. Furthermore, when p-hydroxybenzoates known as preservative are added, IFN-β will be inactivated as described in Japanese Application Laied-Open (Kokai) Publication No. 176216/84.

From EP-A-0 080 879 a pharmaceutical composition, for example an ointment which comprises an effective amount of human interferon is known. Different stabilisers are used for these compositions and different stabilities of interferon containing preparations depending on the polyols are described. As an example glycerin is described which is comprised within the composition in an amount of 45 wt.% at most.

Similar compositions can be derived from EP-A-0 152 345. Para-hydroxybenzoates are added as stabilizers. Glycerin is added in an amount of 5 to 50%. Preparations with α-interferon are described.

The object of the invention is to develop a composition of IFN-β which is stable when stored at room temperature considering that the composition of IFN-β is to be sold in pharmacies and considering that it must be transported. Therefore, the purpose of this invention is to provide a prescription for a composition of IFN-β which is stable even at room temperature.

### DISCLOSURE OF INVENTION

This invention is a stable gel or ointment formulation of interferon-β containing 70 to 85 wt % of glycerin and p-hydroxybenzoates. A gel or ointment formulation of IFN-β which can be stored for a long time at room temperature and in which IFN-β is not inactivated even if the composition contains various additives and in which the stabilization of IFN-β lasts for long time is obtained by this invention.

### BRIEF DESCRIPTION OF DRAWINGS

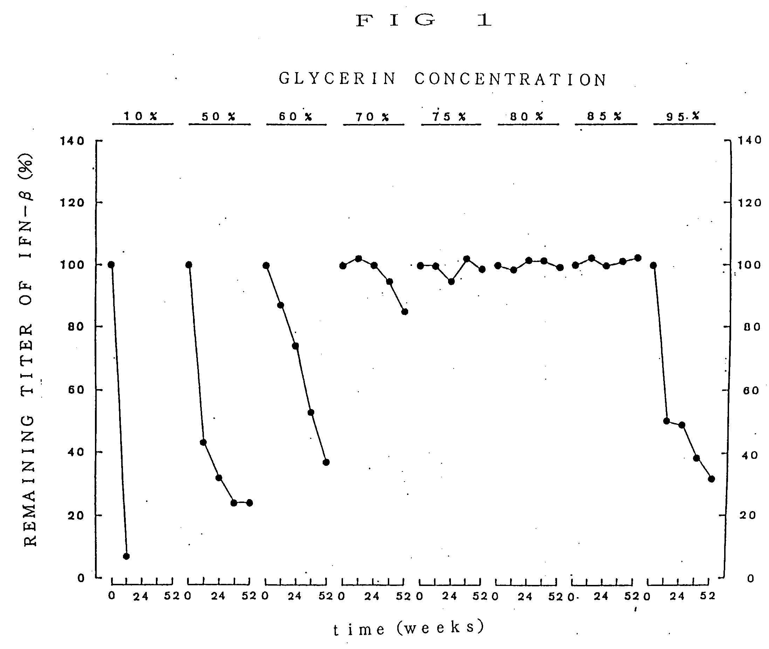

Fig. 1 shows the persistences of the titer of the IFN-β determined in EXAMPLES 1 to 4 and in COMPARATIVE EXAMPLES 1 to 4.

### BEST MODE FOR CARRYING OUT THE INVENTION

In order to obtain a more stable composition of the IFN-β of this invention when the preservatives or the viscosity-increasing agents are added, the composition must contain the glycerin at 70 to 85 wt %.

As for the p-hydroxybenzoates used as the preservatives in this invention, methyl-, ethyl-, propyl-, butylester and so on can be cited. These p-hydroxybenzoates can be employed singly or in combination with two or more types. Ethyl p-hydroxybenzoate and propyl p-hydroxybenzoate are particularly favorable among the above cited p-hydroxybenzoates. The content of p-hydroxybenzoates is usually in the range of 0.01 to 0.2 wt %.

It is favorable that not only glycerin and p-hydroxybenzoates, but also viscosity-increasing agents such as carboxymethyl cellulose and its salts, methyl cellulose, hydroxyethyl cellulose, starch or microfibrous cellulose, and stabilizers such as human serum albumin or human serum globulin, be added when preparing the ointment. As for the viscosity-increasing agents, carboxymethyl cellulose or its salts are favorable, and those in which the viscosity of 1 wt % aqueous solution is (30 - 200 cps) 0.03 - 0.2 Pa·s when measured by a B-type viscometer under conditions of 25 °C and 60 rpm are especially favorable. Specifically, "Daicel 1240", "Daicel 1260", "Daicel 1340", "Daicel 2200" (Daicel Chemical Industries, Ltd.) of the sodium salt of carboxymethyl cellulose are recommended. The content of the viscosity-increasing agents used is usually in the range of 0.1 to 2.5 wt %. As for the stabilizer, human serum albumin or human serum globulin are recommended, but human serum albumin is favored. The content of the stabilizer used is usually in the range of 0.1 to 1 wt %.

Furthermore, the mixing agents, such as the citric acid buffer, can be mixed suitably.

The IFN-β mixed in the composition of this invention can be a compound produced by a cell culture or DNA recombinant technology if it is of human origin. The mixing amount of IFN-β is not specified, but it is favorable to mix IFN-β which has a titer of 1 × 10⁴ IU/g or more.

This invention is further explained showing the embodiments. The measurement of the titer of interferon in the embodiments was performed by the method of cytopathic effect using Sindbis virus, VSV virus and the stabilized cell line of human amnion origin (FL cells) and the obtained values were converted into international units (IU).

### EXAMPLE 1

The IFN-β ["Feron", Toray] of the human diploid fibroblast origin produced by the cell proliferation treatment was used, and the composition of IFN-β is prepared so that the composition contains the following ingredients. The composition ratios are represented as wt %.

| | |
|---|---|
| Glycerin | 70.00 % |
| Ethyl p-hydroxybenzoate | 0.01 % |
| Propyl p-hydroxybenzoate | 0.01 % |
| Sodium carboxymethyl cellulose | |
| "Daicel 1240" | 2.00 % |
| "Daicel 2200" | 0.50 % |
| Human serum albumin | 0.60 % |
| 0.1 M Citric acid buffer (pH 5) | 26.88 % |
| IFN-β | 1×10⁵ IU/g of composition |

The sample of the composition of IFN-β prepared as above described is allowed to remain at 30 °C and the sampling was performed 4, 12 and 24 weeks after preparation. Its titer was determined and the persistence of the titer of the IFN-β was calculated based upon the initial titer of 100%. The obtained results are shown in Fig. 1.

### EXAMPLE 2

The same IFN-β as in Example 1 was used. The composition of IFN-β was prepared so that the composition contains the following ingredients. The composition ratios are represented as wt %.

| | |
|---|---|
| Glycerin | 75.00 % |
| Ethyl p-hydroxybenzoate | 0.01 % |
| Propyl p-hydroxybenzoate | 0.01 % |
| sodium carboxymethyl cellulose | |
| "Daicel 1240" | 2.00 % |
| "Daicel 2200" | 0.50 % |
| human serum albumin | 0.60 % |
| 0.1 M citric acid buffer (pH 5) | 21.88 % |
| IFN-β | 1×10⁵ IU/g of composition |

This composition of IFN-β was stored the same as example 1 and the persistence of the titer of the IFN-β was calculated. The results obtained are shown in Fig. 1.

### EXAMPLE 3

The same IFN-β as in Example 1 was used. The composition of IFN-β was prepared so that the composition contains the following ingredients. The composition ratios are represented as wt %.

| | |
|---|---|
| Glycerin | 80.00 % |
| Ethyl p-hydroxybenzoate | 0.01 % |
| Propyl p-hydroxybenzoate | 0.01 % |
| sodium carboxymethyl cellulose | |
| "Daicel 1240" | 2.00 % |
| "Daicel 2200" | 0.50 % |
| human serum albumin | 0.60 % |
| 0.1 M citric acid buffer (pH 5) | 16.88 % |
| IFN-β | 1×10⁵ IU/g of composition |

This composition of IFN-β was stored the same as example 1 and the persistence of the titer of the IFN-β was calculated. The results obtained are shown in Fig. 1.

### EXAMPLE 4

The same IFN-β as in Example 1 was used. The composition of IFN-β was prepared so that the composition contains the following ingredients. The composition ratios are represented as wt %.

| | |
|---|---|
| Glycerin | 85.00 % |
| Ethyl p-hydroxybenzoate | 0.01 % |
| Propyl p-hydroxybenzoate sodium carboxymethyl cellulose | 0.01 % |
| "Daicel 1240" | 2.00 % |
| "Daicel 2200" | 0.50 % |
| human serum albumin | 0.60 % |
| 0.1 M citric acid buffer (pH 5) | 11.88 % |
| IFN-β | 1×10⁵ IU/g of composition. |

This composition of IFN-β was stored the same as example 1 and the persistence of the titer of the IFN-β was calculated. The results obtained are shown in Fig. 1.

### COMPARATIVE EXAMPLE 1

The same IFN-β as in Example 1 was used. The composition of IFN-β is prepared so that the composition contains the following ingredients. The composition ratios are represented as wt %.

| | |
|---|---|
| Glycerin | 10.00 % |
| Ethyl p-hydroxybenzoate | 0.01 % |
| Propyl p-hydroxybenzoate sodium carboxymethyl cellulose | 0.01 % |
| "Daicel 1240" | 2.00 % |
| "Daicel 2200" | 0.50 % |
| human serum albumin | 0.60 % |
| 0.1 M citric acid buffer (pH 5) | 86.88 % |
| IFN-β | 1×10⁵ IU/g of composition |

This composition of IFN-β was stored the same as example 1 and the persistence of the titer of the IFN-β was calculated. The results obtained are shown in Fig. 1.

### COMPARATIVE EXAMPLE 2

The same IFN-β as in Example 1 was used. The composition of IFN-β was prepared so that the composition contains the following ingredients. The composition ratios are represented as wt %.

| | |
|---|---|
| Glycerin | 50.00 % |
| Ethyl p-hydroxybenzoate | 0.01 % |
| Propyl p-hydroxybenzoate sodium carboxymethyl cellulose | 0.01 % |
| "Daicel 1240" | 2.00 % |
| "Daicel 2200" | 0.50 % |
| human serum albumin | 0.60 % |
| 0.1 M citric acid buffer (pH 5) | 46.88 % |
| IFN-β | 1×10⁵ IU/g of composition |

This composition of IFN-β was stored the same as example 1 and the persistence of the titer of the IFN-β was calculated. The results obtained are shown in Fig. 1.

### COMPARATIVE EXAMPLE 3

The same IFN-β as in Example 1 was used. The composition of IFN-β was prepared so that the composition contains the following ingredients. The composition ratios are represented as wt %.

| | |
|---|---|
| Glycerin | 60.00 % |
| Ethyl p-hydroxybenzoate | 0.01 % |
| Propyl p-hydroxybenzoate sodium carboxymethyl cellulose | 0.01 % |
| "Daicel 1240" | 2.00 % |
| "Daicel 2200" | 0.50 % |
| human serum albumin | 0.60 % |
| 0.1 M citric acid buffer (pH 5) | 36.88 % |
| IFN-β | 1×10⁵ IU/g of composition. |

This composition of IFN-β was stored the same as example 1 and the persistence of the titer of the IFN-β was calculated. The results obtained are shown in Fig. 1.

### COMPARATIVE EXAMPLE 4

The same IFN-β as in Example 1 was used. The composition of IFN-β was prepared so that the composition contains the following ingredients, but some particle substances remained and this composition did not become homogenate. The composition ratios are represented as wt %.

| | |
|---|---|
| Glycerin | 95.00 % |
| Ethyl p-hydroxybenzoate | 0.01 % |
| Propyl p-hydroxybenzoate sodium carboxymethyl cellulose | 0.01 % |
| "Daicel 1240" | 2.00 % |
| "Daicel 2200" | 0.50 % |
| human serum albumin | 0.60 % |
| 0.1 M citric acid buffer (pH 5) | 1.88 % |
| IFN-β | 1×10⁵ IU/g of composition |

This composition of IFN-β was stored the same as example 1 and the persistence of the titer of the IFN-β was calculated. The results obtained are shown in Fig. 1.

### EXAMPLE 5

The same IFN-β as in Example 1 was used. The composition of IFN-β was prepared so that the composition contains the following ingredients. The composition ratios are represented as wt %.

| | |
|---|---|
| Glycerin | 80.00 % |
| Ethyl p-hydroxybenzoate | 0.01 % |
| Propyl p-hydroxybenzoate sodium carboxymethyl cellulose | 0.01 % |
| "Daicel 1260" | 2.00 % |
| human serum albumin | 0.60 % |
| 0.1 M citric acid buffer (pH 5) | 17.38 % |
| IFN-β | 1×10⁵ IU/g of composition |

The sample of the composition of IFN-β prepared as above described was allowed to remain at 30 °C and the sampling was performed 12, 24, 36, 52 and 60 weeks after preparation. Its titer was determined and the persistence of the titer of the IFN-β was calculated as the initial titer is 100%. The results obtained are shown in Table 1.

### EXAMPLE 6

The same IFN-β as in Example 1 was used. The composition of IFN-β was prepared so that the composition contains the following ingredients. The composition ratios are represented as wt %.

| | |
|---|---|
| Glycerin | 80.00 % |
| Ethyl p-hydroxybenzoate | 0.01 % |
| Propyl p-hydroxybenzoate sodium carboxymethyl cellulose | 0.01 % |
| "Daicel 1340" | 2.00 % |
| human serum albumin | 0.60 % |
| 0.1 M citric acid buffer (pH 5) | 17.38 % |
| IFN-β | 1×10⁵ IU/g of composition |

This composition of IFN-β was stored the same as example 1 and the persistence of the titer of the IFN-β was calculated. The results obtained are shown in Table 1.

**Table 1**

| Stability of EXAMPLE 5 and 6 at 30°C | | | | | | |
|---|---|---|---|---|---|---|
| Sample designation | Remaining titer (%) | | | | | |
| | initial | 12 w | 24 w | 36 w | 52 w | 60 w |
| Example 5 | 100 | 94 | 100 | 104 | 83 | 80 |
| Example 6 | 100 | 97 | 96 | 106 | 90 | 87 |

### INDUSTRIAL APPLICABILITY

The composition of this invention is a gel or ointment formulation of IFN-β which is stable for a long time at room temperature. Furthermore, the composition of this invention is an excellent composition in which IFN-β is not inactivated even if the composition contains various additives and the stabilization of IFN-β lasts for long time.

## Claims

1. A stable gel or ointment formulation of interferon-β containing 70 to 85 wt.% of glycerin and p-hydroxybenzoates.

2. The formulation according to claim 1 wherein the content of p-hydroxybenzoates is 0.01 to 0.2 wt.%.

3. The formulation according to claim 1 wherein the p-hydroxybenzoate is ethyl p-hydroxybenzoate or propyl p-hydroxybenzoate.

4. The formulation according to claim 1 wherein the viscosity-increasing agent and the stabilizer are further contained.

5. The formulation according to claim 4 wherein the content of the viscosity-increasing agent is 0.1 to 2.5 wt.%.

6. The formulation according to claim 4 wherein the viscosity-increasing agent is selected from the carboxymethyl cellulose and its salts, methyl cellulose, hydroxyethyl cellulose, starch or microfibrous cellulose.

7. The formulation according to claim 6 wherein the viscosity-increasing agent is carboxymethyl cellulose or its salts.

8. The formulation according to claim 7 wherein the viscosity of 1 wt% carboxymethyl cellulose aqueous solution is (30 - 200 cps) 0.03 - 0.2 Pa.s when measured by a B-type viscometer under the condition of 25°C and 60 rpm.

9. The formulation according to claim 4 wherein the content of the stabilizer is 0.1 to 1 wt%.

10. The formulation according to claim 4 wherein the stabilizer is human serum albumin or human serum globulin.

11. The formulation according to claim 10 wherein the stabilizer is human serum albumin.

12. The formulation according to claim 1 wherein the interferon-β is of human origin.

13. The formulation according to claim 1 wherein the titer of interferon-β is 1 x 10⁴ IU/g of formulation.

## Patentansprüche

1. Stabile Gel- oder Salbenmischung von Interferon-β, die 70 bis 85 Gew.-% Glycerin und p-Hydroxybenzoat enthält.

2. Mischung nach Anspruch 1, worin der Gehalt an p-Hydroxybenzoat 0,01 bis 0,2 Gew.-% beträgt.

3. Mischung nach Anspruch 1, worin das p-Hydroxybenzoat Ethyl-p-hydroxybenzoat oder Propyl-p-hydroxybenzoat ist.

4. Mischung nach Anspruch 1, worin darüberhinaus das viskositätssteigernde Mittel und der Stabilisator enthalten sind.

5. Mischung nach Anspruch 4, worin der Gehalt des viskositätssteigernden Mittels 0,1 bis 2,5 Gew.-% beträgt.

6. Mischung nach Anspruch 4, worin das viskositätssteigernde Mittel aus Carboxymethylcellulose und ihren Salzen, Methylcellulose, Hydroxyethylcellulose, Stärke oder Mikrofasercellulose ausgewählt ist.

7. Mischung nach Anspruch 6, worin das viskositätssteigernde Mittel Carboxymethylcellulose oder ihre Salze sind.

8. Mischung nach Anspruch 7, worin die Viskosität der wäßrigen Carboxymethylcelluloselösung mit 1 Gew.-% bei der Messung mit einem Viskosimeter vom B-Typ bei 25°C und 60 U/min (30 bis 200 cps) 0,03 bis 0,2 Pas beträgt.

9. Mischung nach Anspruch 4, worin der Gehalt des stabilisators 0,1 bis 1 Gew.-% beträgt.

10. Mischung nach Anspruch 4, worin der Stabilisator menschliches Serumalbumin oder menschliches Serumglobulin ist.

11. Mischung nach Anspruch 10, worin der Stabilisator menschliches Serumalbumin ist.

12. Mischung nach Anspruch 1, worin das Interferon-β menschlichen Ursprungs ist.

13. Mischung nach Anspruch 1, worin der Titer des Interferon-β 1 x 10⁴ IU/g der Mischung beträgt.

## Revendications

1. Formulation en gel ou pommade stable d'interféron-β contenant 70 à 85 % en poids de glycérine et de p-hydroxybenzoates.

2. Formulation selon la revendication 1, dans laquelle la teneur des p-hydroxybenzoates est de 0,01 à 0,2 % en poids.

3. Formulation selon la revendication 1, dans laquelle le p-hydroxybenzoate est le p-hydroxybenzoate d'éthyle ou le p-hydroxybenzoate de propyle.

4. Formulation selon la revendication 1, qui contient en outre un agent augmentant la viscosité et un agent stabilisant.

5. Formulation selon la revendication 1, dans laquelle la teneur de l'agent augmentant la viscosité est 0,1 à 2,5 % en poids.

6. Formulation selon la revendication 4, dans laquelle l'agent augmentant la viscosité est choisi parmi la carboxyméthyl cellulose et ses sels, la méthyl cellulose, l'hydroxyéthyl cellulose, l'amidon ou la cellulose microfibreuse.

7. Formulation selon la revendication 6, dans laquelle l'agent augmentant la viscosité est la carboxyméthyl cellulose ou ses sels.

8. Formulation selon la revendication 7, dans laquelle la viscosité de la solution aqueuse de carboxyméthyl cellulose à 1 % en poids est 0,03 - 0,2 Pa.s. (30 - 200 cps) lorsqu'on la mesure avec un viscosimètre du type B à 25°C et 60 tours/minute.

9. Formulation selon la revendication 4, dans laquelle la teneur de l'agent stabilisant est 0,1 à 1 % en poids.

10. Formulation selon la revendication 4, dans laquelle l'agent stabilisant est l'albumine de sérum humain ou la globuline de sérum humain.

11. Formulation selon la revendication 10, dans laquelle l'agent stabilisant est l'albumine de sérum humain.

12. Formulation selon la revendication 1, dans laquelle l'interféron-β est d'origine humaine.

13. Formulation selon la revendication 1, dans laquelle le titre de l'interféron-β est 1 x 10⁴ UI/g de la formulation.
